# EUROPEAN PATENT APPLICATION

(11) **EP 0 940 472 A1**
(43) Date of publication of application: **08.09.1999**
(21) Application number: 97940417.5
(22) Date of filing: 19.09.1997
(51) Int. Cl.: C12Q 1/02, C12M 1/34

(54) **METHOD AND APPARATUS FOR DETECTING BACTERIA**

(30) Priority: 27.09.1996 JP 25617896; 24.03.1997 JP 8878197; 05.09.1997 JP 24091997
(71) Applicant: Organo Corporation, Tokyo 136-8631 (JP)
(72) Inventor: NOGAMI, Takako, Organo Corp. Central Research Lab., Toda-shi, Saitama 335 (JP)
(74) Representative: VOSSIUS & PARTNER
(86) International application number: JP9703323
(87) International publication number: WO9813515

(57) **Abstract**

A bacterium can be detected and examined with identification thereof by simply and specifically labeling the bacterium itself without resort to a change in color tone due to the metabolism of a fermentable substrate or the like, whereby there is provided an excellent method of detecting a bacterium with a simple operation, a shortened determination time, an improvement in efficiency and an improvement in detection accuracy.

A bacteriophage having the nucleic acid thereof labeled with a fluorochrome is brought into contact with a host bacterium to judge the existence or nonexistence of the specific bacterium by making the most of a phenomenon that the fluorescence intensity of the fluorochrome-labeled nucleic acid in a state of existing in the phage shows a significant difference from the fluorescence intensity of the fluorochrome-labeled nucleic acid in a state of being injected into the bacterium.

## Description

### TECHNICAL FIELD

The present invention relates to a method of detecting a bacterium such for example as *Escherichia coli*, a detector for use therein, etc.

### BACKGROUND ART

Microorganisms including bacteria exist in every environment, and keep a close relation with the living of human beings. However, some of microorganisms cause human or domestic animal diseases. Attention is paid to these pathological microorganisms as precaution against infection therewith of human beings and domestic animals. Bacteria in particular among pathological microorganisms multiply rapidly and are hardly affected by environmental conditions such as low temperatures, high temperatures and dryness, so that they persistently exist around human beings and their populations explosively increase once they are exposed to favorable conditions, thereby causing infectious diseases. It is desired to properly maintain control of bacteria not only in waters that are to be ingested directly into human bodies via foods and drinking water (including tap water), but also in waters with which human bodies come in contact, such as water for use in food production processes, water for use in washing of tableware and the like, rivers, lakes and marshes, the sea, pools, baths, amenity water for use in other landscaping water amenities, and waters that have the chances of contact with human beings, such as sewerage and discharge water.

On these premises, a criterion to the effect that "no coliform bacteria are detected" is stipulated for foods and tap water for use as drinking water, a criterion to the effect that "at most two 10-ml water samples in tubes are coliform bacteria-positive when five such water samples in tubes are incubated" for pool water, while a desirable standard value of "at most 10 cells in 1 ml" is set for water for use in landscaping water amenities and a desirable standard value of "at most 3,000 cells in 1 ml" for general wastewaters.

Additionally stated, infectious diseases attributable to bacteria are most frequently caused by bacteria originating in human beings and animals, and these bacteria are liable to bring about widespread casualties. Accordingly, it is considered very reasonable that an item of examination of the existence or nonexistence of enterobacteria such as *Escherichia coli* and coliform bacteria in particular is adopted as a standard for control of foods, drinking water or amenity water in water amenity facilities.

Meanwhile, such detection of bacteria in foods, drinking water (tap water), pool water or water for use in landscaping water amenities has hitherto been done generally by a cultivation method. Such a conventional cultivation method requires some means by which to distinguish, for example, *Escherichia coli* or coliform bacteria from other general bacteria. In many cases, therefore, use of a culture medium wherein *Escherichia coli* or coliform bacteria can be selectively propagated, or a culture medium wherein a measure is taken to change the color tone thereof or develop a special color as the color of colonies upon propagation is made in a detection method to be used in the course of a test.

Additionally stated, apart from the foregoing method, there has been proposed a specific enzyme-substrate culture medium method wherein a test for *Escherichia coli* or coliform bacteria is simplified, i.e., a method designed in such a way that a sample is incubated in the presence of a substrate for an enzyme singularly contained in *Escherichia coli* or coliform bacteria to develop a characteristic color or fluorescence when positive.

Besides these methods, there also are known a PCR method (polymerase chain reaction method), an ATP determination method, a method using a radioactive isotope or the like (Japanese Patent Laid-Open No. 154,700/1996).

However, the foregoing conventional methods involve the following problems. For example, the foregoing cultivation method is not only time-consuming for propagation of a bacterium by cultivation but also complicated in the method of judgment of whether or not a propagated bacterium is *Escherichia coli* or a coliform bacterium. In other words, the cultivation method requires one day only for estimation of whether or not there is contamination with *Escherichia coli* or coliform bacteria and two days for decision of whether or not a contaminant is *Escherichia coli* or coliform bacteria, and takes a long time for judgment because a test involving special dyeing of cells or the like must be repeated if complete examination is desired to be made.

Thus, detection of bacteria according to the cultivation method takes a long time to involve a fear of a case where casualties occur and spread before results are obtained. Apart from the foregoing, since the cultivation method is ordinarily a method wherein colonies formed on an agar plate or a membrane filter and showing peculiar properties are visually counted for quantitative determination or wherein the number of cells contained in an original sample is calculated from the maximum dilution ratio of a positive sample, judgment of colonies showing a singular form is considerably ambiguous. The method of finding the cell count from the maximum dilution ratio can provide only an approximately estimated value to involve a problem of inaccurate quantitative determination.

The aforementioned specific enzyme-substrate culture medium method is advantageous in that a qualitative test for *Escherichia coli* and coliform bacteria can be carried out in 24 hours. However, this method involves a limit attributed to the way of measurement because not all *Escherichia coli* and coliform bacteria can be detected and a quantitative determination test, if desired, must be done only according to a mode of finding the cell count from a value positive at a maximum dilution ratio.

The PCR method, though sharp in sensitivity, involves a problem that actually harmless dead cells are also detected undistinguishably, and cannot be expected much in respect of quantitative determination as well.

On the other hand, the ATP method, though excellent in the simplicity of operation and the capability of specifically detecting only viable cells, is poor in detection sensitivity (incapable of detection if there are not more than 1,000 cells), is restricted in respect of applicable conditions because the luminosity is liable to be affected by exogenous factors, and involves a problem that bacteria cannot be detected with identification thereof.

The above-mentioned method as disclosed in Japanese Patent Laid-Open No. 154,700/1996 encompasses a direct labeling method wherein use is made of a phage labeled with a radioactive isotope or an enzyme, and a method of determining a protein produced by a reporter gene incorporated into a phage. These methods are excellent in that a given bacterium, if desired to be detected, can be selectively detected using a phage in view of the facts that specific bacteriophages exist for almost all bacteria as hosts there of living under ordinary mild conditions on the earth, and that bacteriophages are so strict in recognition of their respective hosts without substantial adsorption on and junction with bacteria other than their respective host bacteria.

However, this method proposed in Japanese Patent Laid-Open No. 154,700/1996 involves the following very hard-to-solve problems in aspects of the speediness of treatment, the simplicity of treatment operation, the level of detection accuracy, etc. when consideration is given to actual working cases.

More specifically, according to the method using a radioactive isotope as a mode of the former direct labeling method, a signal originating in the radioactive isotope label in the possession of a phage adsorbed on a bacterium cannot be distinguished from a signal originating in the same label in the possession of phages not adsorbed on bacteria, thus necessitating an operation of physical separation of the adsorbed phage from the nonadsorbed phages. Further, even if the separation operation is done, there is a problem that phages adsorbed nonspecifically on bacteria (or on separation means such as a filtration membrane) are counted. Apart from this, there is a case where no accurate determination can be done as a result of bacteriolysis ensuing from multiplication of the phage nucleic acid injected into bacterial cells through adsorption of the phage on the bacterial cells. On the other hand, the method using an enzyme as another mode of the direct labeling method involves not only the same problems as in the method using a radioactive isotope, but also a problem of extreme difficulty in actually using any way of detection for accurate determination of a trace amount of the enzyme from a phenomenon developed in a substrate because it is a method wherein use is made of the activity of the trace amount of the enzyme.

On the other hand, a wide use of the latter method using a reporter gene can hardly be expected because a reporter gene in the form of a gene recombinant for every phage specific for every corresponding bacterium must be prepared and a phage nucleic acid including this reporter gene must be subjected to replication, transcription and translation while at the same time requiring an operation of detecting the produced protein.

As described above, all the conventional methods involve the respective problems. Thus, there are problems yet to be solved particularly in aspects of the speediness, the simplicity of operation, the accuracy of detection, etc.

The inventor of the present invention has solved the problems of the foregoing prior art methods to complete the present invention according to which simple and specific labeling of a bacterium itself becomes possible without resort to a change in color tone, for example, due to metabolism of a fermentable substrate, whereby bacteria can be detected and examined with identification thereof to attain great improvements in respect of simple operation, shortened measurement time, efficiency and detection accuracy.

### DISCLOSURE OF THE INVENTION

The instant application provides inventions as set forth in respective claims in the foregoing "claims" column for the purpose of attaining the foregoing object.

The bacterium detection method of the invention of claim 1 of the instant application is characterized by comprising bringing a bacteriophage having the nucleic acid thereof labeled with a dye or a fluorochrome into contact with a host bacterium, and detecting the bacterium having the dye-labeled nucleic acid or fluorochrome-labeled nucleic acid injected thereinto with an optical detection means.

The bacterium detector of the invention of claim 15 of the instant application is characterized by comprising a means for feeding (adding) a solution containing a bacteriophage having the nucleic acid thereof labeled with a dye or a fluorochrome and having a specific adsorbability on a bacterium as an object of detection to a sample liquid (water sample), and an optical detection means for detecting the bacterium having the dye-labeled nucleic acid or fluorochrome-labeled nucleic acid injected thereinto.

Additionally stated, in the following description, the term "fluorochome or the like" as used herein is intented to be a generic term encompassing both a dye and a fluorochrome, while the term "fluorochrome-labeled nucleic acid or the like" as used herein is intended to be a generic term encompassing both a dye-labeled nucleic acid and a fluorochrome-labeled nucleic acid.

In the foregoing description, the bacteriophage is generally a DNA phage having genes constituted of a DNA (single-stranded or double-stranded), but is not intended to exclude an RNA phage having genes constituted of an RNA. Thus, the fluorochrome-labeled nucleic acid or the like may be either a DNA or an RNA. The DNA phage or RNA phage having the nucleic acid labeled with a fluorochrome or the like can be obtained by propagating a bacterium infected with the bacteriophage in a culture medium to which the fluorochrome or the like (dye or fluorochrome) having an affinity for the phage nucleic acid (hereinafter referred to as the "phage DNA") is added. Any substances capable of being bonded to a phage DNA and dyeing or staining a bacterium through injection thereof into the bacterium via the phage can be used as the fluorochrome or the like without any particular limitation. Examples of such dyes include ethidium bromide and propidium iodate, while examples of such fluorochromes include 4,6-diamidino-2-phenylindole hydrochloride (DAPI). Among such fluorochromes, those having an absorption band in a long wavelength range can also be used as dyes. Use of a phage labeled with such a dye enables detection to be done using a microscope, a spectrophotometer or the like instead of using a fluorescence detector such as a fluorescence microscope.

In using either a fluorochrome or a dye in the method of labeling a phage nucleic acid with a fluorochrome or the like, the fluorochrome or the like having an affinity for the nucleic acid (DNA or RNA) may be physically put into spaces in the stereostructure of the nucleic acid by making the most of the affinity, or a nucleotide labeled with the fluorochrome or the like may alternatively be introduced into the phage nucleic acid in the course of synthesis thereof to label it by chemical bonding.

It will suffice that a phage solution for use in detection has a phage concentration capable of allowing at least one particle of the bacteriophage to come into contact with a bacterium contained in a sample liquid in a predetermined time. The level of that concentration can be easily determined experimentally though it cannot indiscriminately be determined because it varies depending on the level of cell count of the bacterium contained in a sample liquid to be subjected to detection (drinking water, amenity water, etc. as mentioned hereinbefore), the detection time, etc. Speaking by way of example, that concentration may as well be set at a level of about 10⁵ to 10¹⁴ particles, preferably 10⁷ to 10¹⁴ particles, most suitably 10⁸ to 10¹² particles, per ml of the sample liquid (water sample) in many cases where *Escherichia coli* contained in drinking water is to be detected. An increase in the phage concentration of the phage solution can favorably increase the probability of contact of a plurality of phage particles with a bacterium not only to increase the dyeing density of the dye for a single bacterium being detected but also to enhance the intensity of fluorescence emitted from a single bacterial cell, whereby higher-accuracy detection can be done. When the phage concentration is lower than 10⁵ particles/ml, the probability of contact of the phage with the bacterium is so low as probably to entail a long time for determination or to lead to a failure in detection even if the bacterium exists. On the other hand, when the phage concentration is increased to exceed 10¹⁴ particles/ml, the viscosity of a sample liquid is increased and the intensity of background fluorescence is enhanced to pose a problem of lowering the sensitivity of measurement. Thus, it may as well be set to be in the above-mentioned range.

In general, the process of infection with a bacteriophage (bacteriolytic infection or lysogenization) comprises 3 divided stages, i.e., adsorption and junction thereof onto a host bacterium, injection of the phage DNA into a host cell, and replication of phage particles with the injected phage DNA as a template.

A phage having the fluorochrome-labeled nucleic acid or the like is used in the present invention for the following reasons: Specifically, while a bacteriophage is adsorbed specifically on the host bacterium as described above, the fluorochrome-labeled nucleic acid or the like to be used in the present invention is inferred to be transformed by unfolding and spreading in a bacterial cell wherein it has been injected, as against a state of being densely packed in the phage, when comparison is made between the fluorochrome-labeled nucleic acid or the like existing in the phage and that injected in the bacterial cell, with the result that an optically distinguishable significant difference is caused in the detected intensity of fluorescence as confirmed, for example, in the case of a fluorochrome in Referential Examples 1 and 2, which will be described later. While paying attention to this finding, the inventor of the present invention has completed the present invention characterized in that only a bacterium having a phage DNA injected thereinto are optically distinguished and detected without need of any operation of physical separation of phage particles not adsorbed on bacteria by making the most of such a difference.

Another feature to which attention should be paid in the present invention is that it has been noticed that the process of injection of a phage DNA into cells of a host bacterium by phage particles adsorbed on the bacterial cells depends on the energy state of the bacterial cells of the host in many cases. More specifically, the phage DNA (nucleic acid) is not injected into host cells which have lost their biological activity, but only into living cells (viable cells). In other words, viable cells can be optically detected since the phage DNA is injected thereinto, while optical information on dead cells or fragments having no phage DNA injected thereinto despite adsorption thereon of the phage DNA can be definitely distinguished. The function of the present invention that viable cells are discerned from dead cells by making the most of a phenomenon that whether or not there are any activity and injection of the phage is optically discernible, cannot be expected according to the conventional methods using a radioactive isotope, an enzyme or the like, and is very advantageous in aspects of simplification of treatment operations and an improvement in detection accuracy.

Even if a phage is adsorbed nonspecifically on a bacterium, the phage DNA is not injected thereinto. Thus, the method of the present invention, wherein only a bacterium having a phage DNA injected thereinto is detected, is excellent in an aspect of a capability of high-accuracy detection.

Further, transcription of any genes in a phage DNA labeled with a fluorochrome or the like is inhibited under ordinary conditions because the activity of the genes is lost through labeling of the DNA with the fluorochrome or the like. This is one of the functions of the present invention to which attention should be paid. More specifically, the phage DNA labeled with the fluorochrome or the like to be used in the present invention, even if injected into bacterial cells, is usually incapable of replication of any new phage particles and formation of a group of enzymes for burst of newly formed phage particles out of the bacterial cells through destruction of the host cells, therefore giving rise to no bacteriolysis of the host cells as otherwise recognized in the last stage of the infection process. This allows the phage DNA labeled with the fluorochrome or the like to be maintained stably in the host cells, thus providing an advantage that the treatment time, either long or short, little affect the detection accuracy. Additionally stated, where there is a possibility that transcription of the phage nucleic acid occurs depending on the kind of bacterium, the environmental conditions, the kind of used fluorochrome or the like, etc., any treatment may be carried out for positively preventing the transcription and replication. For example, either addition of a DNA synthesis inhibitor or a protein synthesis inhibitor to a sample liquid, or alteration of part of a phage gene to a nonsense codon will suffice for loss of the capability of transcription and replication.

For the foregoing reasons, only viable cells of a given bacterium can be detected according to the present invention by dyeing or staining them fluorescent using a bacteriophage having the nucleic acid thereof labeled with a fluorochrome and having a specific adsorbability thereon.

A bacterium dyed with a dye or stained with a fluorochrome through adsorption thereon of a bacteriophage having the fluorochrome-labeled nucleic acid or the like and injection thereinto of the nucleic acid can be detected by an optical means. Usable examples of the optical means, though not particularly limited in the type or the like in so far as capable of detecting the dye or the fluorescence, include the ordinary microscope and spectrophotometer in the case of dye labeling, and a fluorescence microscope, a fluorometer, and a fluorescence detector in the case of fluorochrome labeling. Alternatively, use may be made of a method wherein detection of dye points, fluorescence points or the like is done through image analysis of a detected image, a method wherein a difference in the intensity of fluorescence is detected using a fluorometer, a method wherein the number of dyed particles or fluorescent particles in a flowing liquid is detected using a flow cell, or the like.

When a fluorochrome is used as a label, a case where a fluorescence detector with a sharp sensitivity is adopted can be mentioned as the simplest case. For example, a fluorochrome-labeled phage solution and nutrient salts are fed (added) to a sample liquid, whereby whether or not a specific bacterium exists therein can be simply examined based on whether or not an increase, even if slight, in the intensity of fluorescence is recognized after the lapse of predetermined time. Alternatively, nutrient salts and an excessive amount of a phage solution may be added to a sample liquid containing bacteria, and the resulting mixture may be centrifugalized or membrane-filtrated for bacteria-phage separation after the lapse of predetermined time to obtain a bacteria fraction, the fluorescence intensity of which is then measured, whereby whether or not a bacterium exists therein can be simply examined.

While using the foregoing method and device, for example, a specific phage labeled with a dye or a fluorochrome for each of a variety of bacteria may be prepared, and may then be brought into contact with the bacterium. In this case, that bacterium, even if unknown in respect of what species it is, can be identified in such a simple manner as to be *Escherichia coli* if dyed with an *Escherichia coli*-specific phage or to be *Salmonella* if dyed with a *Salmonella*-specific phage. This is advantageous in that the identification test can be carried out in a short time (in one hour) for certain as compared with a conventional method wherein the degradability of a fermentable substrate is examined (requiring at least 2 days).

Cases where the identification of a bacterium is particularly required include a case where a bacterium causative of a microbism such as a sitotoxism or a nosocomial infection in a hospital is identified, and a case where bacteria as contaminants in water or the like for use in a food factory or the like are identified. Specifically speaking, there can be mentioned, for example, a case where bacteria, the existence of which is to be avoided, are detected in environmental water, environmental air, foods, city water, sewerage, hospitals (particularly at clinical sites), precision apparatus manufacturing factories, etc. wherein bacteria are required to be detected. Examples of principal combinations of bacteria to be avoided and their specific phages include *Escherichia coli* and T phage, λ phage, or the like; *Salmonella* and P phage or the like; *Pseudomonas* and P phage or the like, *Klebsiella* and Stanford University 60 or 92 phage; *Clostridium* and 70, 71 or 72 phage; *Shigella* and φ 80, or Stanford University 37 or D20 phage; *Corynebacterium* and C phage; and *Micrococcus* and N phage or ML53-40 phage. In this case, a concentration step must often be taken in order to detect a trace amount of a bacterium.

Further, the present invention can be effectively used for detecting useful bacteria. More specifically, in the fields where foods, medicines, etc. are produced using microorganisms (bacteria), the viable cell count of any active bacterium can be confirmed using the method of the present invention wherein only viable cells can be counted. This enables the method to be used, for example, for control of the amount of active yeast in a beer brewing process, control of the amount of active lactic acid bacteria in a yogurt production process, and confirmation of the activity of microorganisms capable of producing amino acids or the like. Additionally stated, in many cases where any useful bacterium is to be detected, the step of diluting a solution having a high bacterium concentration at a suitable dilution ratio must often be taken.

As for conditions of infection of a bacterium with a phage, the temperature under aerobic conditions, though dependent on the kind of bacterium, is set to be 0 to 120 °C for thermophilic bacteria and to be 50 °C or lower for general bacteria and phages, which are not deactivated at these temperatures. In these ranges, the higher the temperature, the higher the rate of infection. A sample liquid is also preferably stirred to enhance the efficiency of contact of a bacterium with a phage.

The sample liquid from which any bacterium is to be detected can be subjected as such to detection when it is a water-based sample. When the sample liquid is a liquid sample containing an oil, it can be subjected to detection like a water-based sample after dispersion thereof with a nonionic surfactant if the oil content thereof is low, but a water phase obtained by addition thereto of water for extraction of a bacterium into the water phase may be subjected as a sample liquid to detection like a water-based sample if the oil content is high. When a sample is solid, water is added to the sample, which is then ground down and then stripped of the solid by centrifugalization, filtration or the like to obtain the supernatant, which can be subjected as a sample liquid to detection like a water-based sample. When the sample liquid contains a dye or a fluorochrome, detection is preferably done after dialysis using physiological saline for removal of the dye or the fluorochrome. When concentration of the sample liquid with a microfilter is necessary, there may be adopted a method wherein it is replaced with a liquid obtained by a liquid exchange method comprising such concentration and subsequent addition of physiological saline to the same concentration tank.

The existence or nonexistence of a bacterium as well as the cell count thereof can be examined, for example, according to a method comprising counting as bacterial cells points where the intensity of fluorescence exceeds a given value on the basis of information detected with an optical means. A device therefor can be constructed in accordance with an image processing technique using a microcomputer (MPU) or the like.

The detection method of the invention of claim 2 of the instant application is characterized by comprising capturing bacteria contained in air or a liquid by a capture means such as filtration or adsorption, bringing a bacteriophage having the nucleic acid thereof labeled with a dye or a fluorochrome into contact with the captured bacteria, and detecting a bacterium having the dye-labeled nucleic acid or fluorochrome-labeled nucleic acid injected thereinto with an optical detection means. The bacterium detector of the invention of claim 16 is characterized by comprising a capture means for capturing bacteria contained in air or a liquid on a membrane, a phage solution contact means for bringing a phage solution containing a bacteriophage having the nucleic acid thereof labeled with a dye or a fluorochrome into contact with the captured bacteria, and an optical detection means for detecting a bacterium having the dye-labeled nucleic acid or fluorochrome-labeled nucleic acid injected thereinto.

Examples of the above-mentioned capture means include activated carbon; and filtration membranes such as a hollow fiber membrane, a microfilter, an ultrafilter, and a reverse osmosis membrane. Usable examples of adsorption means include a nonwoven fabric, a fibrous filtering material, an ion exchange resin, a sand filtering material, etc., which are capable of adsorbing thereon bacteria.

According to this invention, water to be treated is passed across a filter for filtration, set, for example, in water treatment equipment, to capture bacteria on the filter, which is then either directly brought into contact with a phage solution, or immersed in physiological saline or water containing nutrient sources and then stripped of the bacteria with such ultrasonic waves as not to destroy the bacteria to obtain the supernatant, which is then fed as a sample liquid with a phage solution, whereby a bacterium in water to be treated can be detected and identified. Further, since the bacterium is optically detected after captured on a filtration means such as a membrane, sure detection of the bacterium is possible. This is therefore suitably applicable to a case where high-accuracy simple detection is desired though not particularly required to be done comparatively quickly.

Additionally stated, the method wherein a phage solution is added to a liquid phase after bacteria attached to a membrane is stripped off thereinto can provide a high probability of a bacterium encountering a phage in the liquid phase, and is therefore preferred to a case where bacteria attached to a membrane is brought as such into contact with a phage solution.

The method of the present invention can be used not only for the foregoing detection of a bacterium in a sample liquid (water sample) but also for detection of a floating bacterium in air. For example, a gas (air) containing bacteria floating therein may be diffused (bubbled) into water to trap the bacteria in water, which is then used as a sample liquid. Sucked air may alternatively be filtrated with a filter to capture bacteria on the filter in substantially the same manner as described above, and the filter may then be either directly brought into contact with a phage solution, or immersed in physiological saline or the like and then stripped of the bacteria to obtain the supernatant, which is then fed with a phage solution. The foregoing procedure can be suitably applied to examination of the state of an environmental space in a hospital, a food factory, or the like, wherein bacterial contamination becomes a problem, for example, through inspection of a filter set in an air conditioner or other air purification facilities.

The detection method of the invention of claim 3 of the instant application is characterized by comprising concentrating a bacterium to be detected in a sample liquid containing the bacterium low in concentration, feeding a bacteriophage having the nucleic acid thereof labeled with a fluorochrome or the like, for which the bacterium is a host, into the resultant liquid containing the bacterium concentrated therein to effect contact thereof with the host bacterium, and detecting the bacterium having the fluorochrome-labeled nucleic acid or the like injected thereinto with an optical means. The detector of the invention of claim 17 is characterized by comprising a means for concentrating a bacterium in a sample liquid, a phage solution feed means for feeding a phage solution containing a bacteriophage having the nucleic acid thereof labeled with a fluorochrome or the like to the resultant concentrated sample liquid, and an optical detection means for detecting the bacterium having the dye-labeled nucleic acid or fluorochrome-labeled nucleic acid injected thereinto.

In the foregoing cases, concentration is done using, for example, a membrane, which may be used without any particular restriction of the kind and material of membrane in so far as the membrane can inhibit passage thereacross of a bacterium as an analyte. Additionally stated, when a sample liquid containing an analyte is filtered, pre-filtration is preferably done using a membrane capable of allowing the bacterium to be passed thereacross but inhibiting passage thereacross of SS larger than it. Specific examples of the device for use in such a bacterium concentration method include a device wherein a given amount of, for example, flowing sample water is automatically sampled at given intervals and put into a tank having a membrane, wherein it is then automatically concentrated to obtain the concentrated sample water, which is then automatically withdrawn for automatic measurement thereof.

According to this invention, a thin population of the bacterium present in the sample solution can be effectively detected after concentration.

The foregoing concentration of the bacterium may be done according to either an embodiment wherein a liquid is concentrated as a whole to concentrate the bacterium, or an embodiment wherein the bacterium is localized in a liquid for partial concentration thereof. The invention of claim 4 of the instant application is characterized by feeding a bacteriophage having the nucleic acid thereof labeled with a fluorochrome or the like into a liquid wherein a bacterium is concentrated by membrane separation or centrifugalization.

Other methods of concentrating a bacterium include one wherein an attractant to a given bacterium to be detected may be fed in a small zone in a sample liquid as in the invention of claim 5, and one wherein such an attractant may be fed using a capillary as in the invention of claim 6. Examples of the attractant include amino acids such as serine and aspartic acid; saccharides such as glucose, galactose, maltose, and ribose; and organic acids such as citric acid and malic acid. Besides the above-mentioned organic substances, there can be mentioned oxygen, fumaric acid, nitric acid, etc. all capable of becoming an electron acceptor in a respiratory chain.

Specific examples of the method of concentrating a bacterium by making the most of an attractant include the following methods: (1) A liquid containing both an attractant such as glucose or serine and a phage having the nucleic acid thereof labeled with a fluorochrome or the like is fed to a solution under test while using a capillary, whereby the bacterium is concentrated in the vicinity of the tip of the capillary to be brought into contact with the phage. (2) An attractant is fed to a solution under test while using a capillary to confirm concentration of a bacterium in the vicinity of the tip of the capillary with a microscope or the like, followed by feeding thereto the above-mentioned phage using another capillary. (3) The above-mentioned phage is fed to a solution under test, followed by feeding thereto an attractant using a capillary.

The invention of claim 7 of the instant application is characterized by comprising diluting a sample liquid thickly containing a bacterium to be detected, feeding a bacteriophage having the nucleic acid thereof labeled with a dye or a fluorochrome, for which the bacterium is a host, to the diluted sample liquid containing the bacterium to bring it into contact with the host bacterium, and detecting the bacterium having the dye-labeled nucleic acid or fluorochrome-labeled nucleic acid injected thereinto with an optical means. Any appropriate diluent feed unit may be used for dilution of the sample liquid.

According to this invention, the viable cell count in a solution thickly containing a bacterium can be detected, or measured. Thus, this can be utilized for control of bacterial activity, for example, in a food production process or the like wherein a useful bacterium is used.

The invention of claim 8 of the instant application is characterized by comprising bringing a bacteriophage having the nucleic acid thereof labeled with a dye or a fluorochrome with a host bacterium in a sample liquid, and detecting the bacterium having the dye-labeled nucleic acid or fluorochrome-labeled nucleic acid injected thereinto according to flow cytophotometry. The detector of the invention of claim 21 is characterized by comprising a liquid feed means for continuously passing a sample liquid through a flow cell, a phage solution addition means for adding a phage solution containing a bacteriophage having the nucleic acid thereof labeled with a dye or a fluorochrome to the sample liquid in front of the flow cell, and an optical detection means for detecting the bacterium having the dye-labeled nucleic acid or fluorochrome-labeled nucleic acid injected thereinto in the flow cell.

These inventions enable the number of fluorescent particles, i.e., the cell count, in a flowing solution passing through the flow cell to be detected, whereby the on-line real-time detection thereof can be done continuously.

The detection method of the invention of claim 9 of the instant application is characterized in any one of the foregoing method inventions by feeding a nutrient source, necessary for adsorption and nucleic acid injection in the initial stage of infection of a host bacterium with a bacteriophage, to the host bacterium. The detector of the invention of claim 23 is characterized by comprising a nutrient source feed means for feeding a nutrient source, necessary for adsorption and nucleic acid injection in the initial stage of infection of a host bacterium with a bacteriophage, to a sample liquid.

The foregoing operation is unnecessary when nutrient salts and the like (carbon source in particular) already sufficiently exist in a sample liquid. In the case where nutrient salts are insufficient, however, no metabolism of a bacterium results in poor infection, which in turn may sometimes involve no injection of a phage DNA into the bacterium. This can be eliminated by the above-mentioned inventions.

The invention of claim 11 of the instant application is characterized in each of the foregoing inventions by comprising labeling the nucleic acids of different kinds of phages with respective dyes differing in hue or respective fluorochromes differing in excitation wavelength or fluorescence wavelength, and adding them to a sample liquid containing a plurality of bacteria on which these different kinds of phages are respectively adsorbed specifically, whereby that plurality of bacteria are detected simultaneously.

According to the method of this invention, phages having the respective nucleic acids thereof labeled with respective fluorochromes differing in excitation wavelength or fluorescence wavelength and having respective specific adsorbabilities on bacteria such for example as *Escherichia coli*, *Salmonella*, and other bacteria are used for a liquid containing these bacteria coexistent therein or the like, whereby the operation of detecting these bacteria can be done simultaneously and collectively.

The invention of claim 12 of the instant application is characterized in each of the foregoing inventions by labeling the nucleic acids of different kinds of phages with the same dye or the same fluorochrome, and adding them to a sample liquid containing a plurality of bacteria on which these different kinds of phages are respectively adsorbed specifically, whereby the total cell count of that plurality of bacteria are detected.

According to the method of this invention, for example, whether or not a plurality of bacteria to be examined exist in a liquid or the like as the object can be easily examined.

The methods of the foregoing inventions are effective in detecting and identifying pathogenic bacteria particularly in a hygienic test or the like. For example, pathogenic enterobacteria principally causative of food poisoning, or sitotoxism, examples of which include *Escherichia coli*, coliform bacteria, dysentery bacilli, cholera bacilli, salmonellae, vibrios, botulinus bacilli, *Clostridium welchii*, staphylocci, and *Bacillus cereus*, have the respective nucleic acids thereof labeled with the same or different dye(s) or fluorochrome(s), whereby these bacteria can be easily detected using phages for which the bacteria are hosts.

The foregoing inventions are also applicable to bacteria causative of infectious diseases other than food poisoning, examples of which include tubercle bacilli, pneumococci, *Pseudomonas aeruginosa*, *Legionella*, and other bacteria, and can also be effectively used in a confirmation test wherein a colony of a bacterium is collected and suspended in a phage solution to identify it by whether or not it is stained fluorescent.

The invention of claim 13 of the instant application is characterized in each of the foregoing method inventions by comprising measuring the intensity of fluorescence from or the size of light source of fluorescence from a bacterium having a fluorochrome-labeled nucleic acid injected thereinto, and comparing the measured value thereof with a predetermined threshold value, whereby every light source in excess of the threshold value is regarded as the bacterium detected. On the other hand, the invention of claim 14 is characterized in that the existence or nonexistence of a bacterium or the cell count thereof is detected from an image after image processing as to the intensity of fluorescence or the size of light source measured with an optical detection means according to an image processing procedure of excluding light sources having at most a predetermined threshold value of the intensity of fluorescence or the size of light source. Further, the invention of claim 21 is characterized in that each of the devices of the foregoing inventions is provided with an image pickup means as an optical detection means and an image processing means for excluding light sources having at most a predetermined threshold value of the intensity of fluorescence or the size of light source from a picked-up image obtained by the image pickup means.

According to these inventions, for example, the size of light source, when a fluorochrome is injected into a bacterium, can be distinguished with a significant difference from the size of light source, when the fluorochrome exists in a phage. Thus, on the basis of the predetermined threshold value that is smaller than the former but larger than the latter as an index indicative of the size such as an area or a length, the existence or nonexistence and cell count of the bacterium can be computed. When the picked-up image is subjected to such image processing as to exclude light sources equal to or smaller than the threshold value in size, an image of only a bacterium emitting a fluorescence can be obtained to enable high-accuracy detection and also to be effective in automatizing the detection. Additionally stated, image processing "excluding" light sources equal to or smaller than the threshold value in size is intended to mean that light sources having, for example, at most the threshold value are regarded as the same as the background. The object of comparison with the threshold value is not limited to the size of light source, and comparison may be done using the intensity of fluorescence of light source in the same manner.

The portable bacterium detection kit of the invention of claim 24 or 25 of the instant application is characterized by comprising a bacterium detection aid containing a carbon source capable of being assimilated by a bacterium as the object of detection, a bacteriophage reagent having the nucleic acid thereof labeled with a dye or a fluorochrome, and a reaction vessel prepared for addition thereto of a sample as the object of measurement.

The above-mentioned bacterium detection aid and bacteriophage reagent may be prepared, for example, in respective containers filled therewith.

The above-mentioned bacterium detection aid comprises at least a carbon source capable of being assimilated by the bacterium. Usable examples of such a carbon source include carbohydrates such as saccharides including monosaccharides such as glucose, disaccharides and trisaccharides. Addition may also be made of tryptophan or the like capable of improving the adsorbability of a phage. According to this invention, since no labor for preparing materials is necessary, efficient work can be done in laboratories and the like having no facilities necessary for such detection. Further, the above-mentioned kit may be combined with a portable optical detection means. Examples of the portable optical means include miniature microscope, spectrophotometer, and fluorophotometer.

In this case, outdoor detection work can be done on site, and the work can also be done efficiently.

Additionally stated, the present invention is not limited to the foregoing constitutions of methods and devices, and may have other constitutions added thereto if necessary. For example, tryptophan, bivalent cations of magnesium, calcium or the like, or the like may be added as an aid in order to improve the adsorbability of the phage on the bacterium.

### BRIEF DESCRIPTION OF THE DRAWINGS

Fig. 1 is a flow diagram illustrating the schematic constitution of equipment according to Embodiment 1 of the present invention.
Fig. 2 is a flow diagram illustrating the schematic constitution of equipment according to Embodiment 2 of the present invention.
Fig. 3 is a flow diagram illustrating the schematic constitution of equipment according to Embodiment 3 of the present invention.
Fig. 4 is a diagram illustrating the schematic constitution of equipment according to Embodiment 6 of the present invention.
Fig. 5 is a diagram illustrating the outline of operations for applying the method of Embodiment 7 of the present invention.
Fig. 6 is a diagram illustrating a state when the method of Embodiment 8 of the present invention is applied.
Fig. 7 is a diagram illustrating a state resulting from application of the method of Embodiment 7 of the present invention.
Fig. 8 is a diagram illustrating the outline of constitution when the method of Embodiment 9 of the present invention is applied.
Fig. 9 (a) is a microscopic photograph showing a state of detection of fluorescence from a phage having the nucleic acid thereof labeled with a fluorochrome and from a bacterium stained by injection thereinto of that labeled nucleic acid of the phage, while Fig. 9 (b) is a diagram after image processing of that microscopic photograph.

### Explanation of Numerals

1 ... line, 2, 6 ... reactors, 3 ... detector, 4 ... concentrated culture medium, 5 ... phage solution, 7 ... filter, 41, 51 ... pumps, 52 ... flow meter, 60 ... sample liquid, 61 ... light source for excitation of fluorochrome, 62 ... image pickup tube device, 63 ... image processing unit, 201 ... water sample, 202 ... slide glass, 203 ... slide glass holder, 204 ... lens of fluorescence microscope, 205 ... stage of fluorescence microscope, 206 ... capillary, 207 ... lens of inverted microscope.

### MODES FOR CARRYING OUT THE INVENTION

The foregoing detection methods and detectors for applying them will now be described.

### Embodiment 1

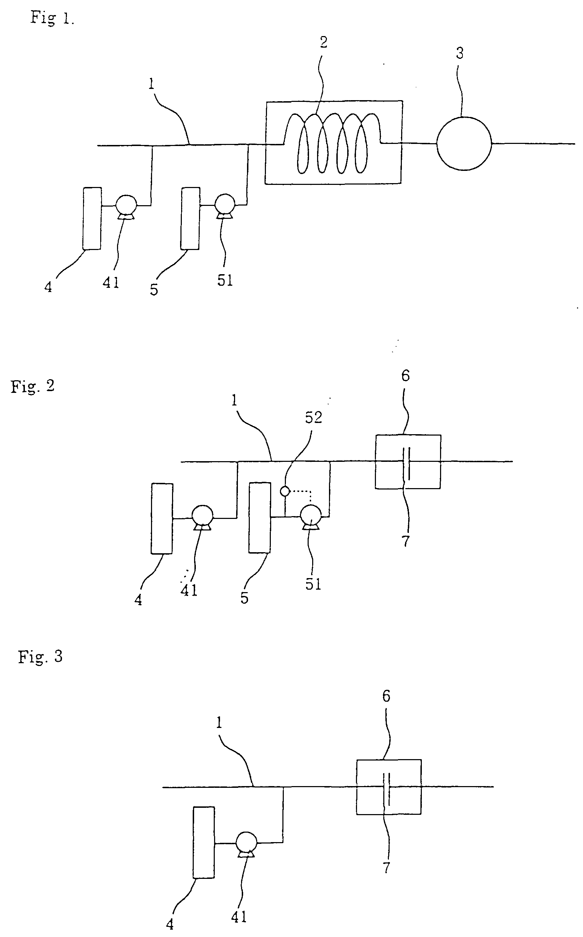

Fig. 1 is a schematic flow diagram of an example of equipment for applying a method of the present invention. In this figure, numeral 1 denotes a line through which a sample liquid (hereinafter referred to as an "water sample") from which a bacterium is to be detected is passed, and which is sequentially connected, on the downstream side thereof, to a coiled reactor 2 for stirring a liquid comprising the water sample and a phage solution added thereto, and then to a fluorescence detector 3 for detecting the bacterium. On the other hand, on the upstream side of the reactor 2, the line 1 is connected to a pump 41 for feeding a concentrated culture medium (nutrient source solution) 4 containing amino acids, saccharides, inorganic salts, etc. and separately to a pump 51 for feeding the solution (hereinafter referred to as the "phage solution") 5 containing a phage having the nucleic acid thereof labeled with a fluorochrome or the like (dye or fluorochrome).

A description will be made of the procedure of detecting the bacterium using the equipment of this example. The concentrated culture medium 4 and the phage solution 5 are continuously added by means of the respective pumps 41 and 51 to the water line 1 through which the water sample from which the bacterium is to be detected is passed.

The water sample to which the above-mentioned liquids are added are mixed with these two added liquids while flowing through the line 1, and enters the coiled reactor 2 maintained at 37 °C as an optimum environment for a microorganism reaction. When the specific bacterium exists in the water sample, the phage reacts with the specific bacterium, whereby the phage nucleic acid (e.g.; phage DNA) labeled with the dye or the fluorochrome is injected into cells of the bacterium.

The bacterium coming out of the reactor 2 is sent to a detector 3 such as a microscope or spectrophotometer for detecting the dye, or a fluorescence detector for detecting the fluorescence to detect there the specific bacterium stained, or dyed, with the fluorochrome or the like by injection thereinto of the phage nucleic acid. The mode of the detector 3 may be either of a type wherein stained particles in a stationary sample liquid are detected like a microscope or a fluorescence microscope, or of a type wherein stained particles flowing through a flow cell are detected. In either case of the case of stationary detection and the case of detection in a flowing state, use may be made of a detector of a type wherein an image pickup tube device such as a CCD camera is used to picture an image of a sample liquid from which photoelectrically transferred signals are obtained.

Alternatively, photoelectrically transferred signals obtained through detection with the detector 3 in the form of an image pickup tube device may be inputted into a computing unit such as an MPU (not shown in the figure) wherein a program for examination of the existence or nonexistence, or quantitative determination of a given bacterium is incorporated, whereby the existence or nonexistence of the bacterium and the cell count thereof may be detected.

Additionally stated, use of a fluorescence detector of such a type as a flow cytometer with which the number of fluorescent particles is counted in a flow cell permits designing a measuring instrument capable of continuous detection of the cell count in line water involved in production or treatment of city water, sewerage, drinking water, a soft drink, or the like. The term "flow cytometer" indicates a device for use in flow cytophotometry for measuring the number of fluorescent particles passing through a flow cell. This device can measure and indicate the number of particles for every class of intensity of fluorescence.

### Embodiment 2

Fig. 2 is a flow diagram illustrating the schematic constitution of another example of equipment according to the present invention. The feature of this example is such that substantially the same constitution as in Embodiment 1 is adopted except that a reactor 6 having a built-in filter 7 is characteristically used instead of the coiled reactor 2 as shown in Fig. 1. Accordingly, the other constituents are the same as in the foregoing Embodiment 1, and the same constituents are hence denoted by the same numerals with explanations thereof dispensed with.

A description will be made of the procedure of detecting a bacterium using this equipment. The aforementioned concentrated culture medium 4 and phage solution 5 are continuously added by means of the respective pumps 41 and 51 to the water line 1 through which the water sample from which a bacterium is to be detected is passed.

The water sample to which the concentrated culture medium 4 and the phage solution 5 are added, while being mixed with the two added liquids, is passed across the filter 7 built in the reactor 6 maintained at 37 °C. This filter 7 to be used is one capable of capturing bacteria but allowing the phage to be passed thereacross, and is preferably in the range of, for example, 0.2 µm to 0.45 µm in pore size.

When the bacterium as the object of detection exists in the water sample, the phage having the nucleic acid thereof labeled with the dye or the fluorochrome comes into contact with and reacts with the bacterium in the line 1 or on the filter 7 having the bacterium captured thereon to inject the phage DNA into cells of the bacterium. After filtration of a given amount of the water sample, the filter is detached, and is then either observed with a microscope or a fluorescence microscope or examined with other detector capable of measuring the intensity of fluorescence, whereby the existence or nonexistence of the given bacterium and the like can be judged.

Additionally stated, in this example, the flow rate through the feed pump 51 is controlled by feed forward control or feedback control on the basis of the feed rate of the phage solution 5 measured by the flow meter 52. This is so because bacterium detection conditions are preferably set as constant as possible. As for flow rate control, an appropriate choice may be made of a control method of making the flow rate constant, a control method of increasing or decreasing the feed rate in accordance with a change in the excitability and fluorescence-emitting capability of the fluorochrome with time.

### Embodiment 3

Fig. 3 is a flow diagram illustrating the schematic constitution of still another example of equipment according to the present invention. This example is different from the foregoing Embodiment 2 in that the phage solution is not added to the line 1, but is substantially the same as in Embodiment 2 in respect of other constituents. Thus, the same constituents are denoted by the same numerals with explanation thereof dispensed with.

A description will be made of the procedure of detecting a bacterium using this equipment. A concentrated culture medium 4 is added to the water line 1 through which a water sample from which a bacterium is to be detected is passed. A given amount of the water sample is filtrated with a filter 7 in a reactor 6 maintained at 37 °C. In the method of this example, the bacterium captured on the filter 7 is propagated by the culture medium added thereto.

Next, the filter 7 is detached after the lapse of predetermined time. Bacteria captured on the filter 7 is brought into contact with a phage solution (for example, by immersing this filter in the phage solution). After the lapse of predetermined time, the filter is either observed with a microscope in the case of dye labeling or a fluorescence microscope in the case of fluorochrome labeling to count the number of dyed particles, or image-wise picked up with an image pickup tube device.

The bacteria captured on the filter are propagated before detection according to the method of this example, which is therefore effective in a case where, because of only a trace amount of a given bacterium, a large amount of a sample liquid must otherwise be used as the object in order to confirm the existence or nonexistence therein of the bacterium.

### Embodiment 4

The filter 7 having bacteria captured thereon in Embodiment 3 described using Fig. 3 above is immersed in a liquid containing nutrient sources for a bacterium while agitating or centrifugalizing the liquid to remove the bacteria from the filter 7. A phage solution is added to the remaining liquid after removal of the filter 7, followed either by observation with a detector such as a microscope or a fluorescence microscope or by measurement of the intensity of fluorescence with other detector capable of measuring the intensity of fluorescence.

According to this example, since the bacterium is brought into contact with a phage in the liquid phase containing the bacteria removed from the filter, chances of contact of the bacterium with the phage can be given at a high rate with a simultaneous agitation operation or the like, whereby surer bacterium detection is possible as against that in Embodiment 3.

### Embodiment 5

In this example, there is shown a case where the water line 1 in Embodiment 3 described using Fig. 3 above is replaced with a gas flow system wherein environmental air is suctioned and passed to allow the filter 7 to capture bacteria floating in air. In this case, however, the concentrated culture medium 4 and the feed pump 41 are dispensed with.

The filter 7 on which bacteria floating in air are captured can serve for detection of a bacterium floating in air either by direct contact of the filter with the phage solution like in Embodiment 3 or by removal of the bacteria from the filter and subsequent contact thereof with the phage solution like in Embodiment 4.

### Embodiment 6

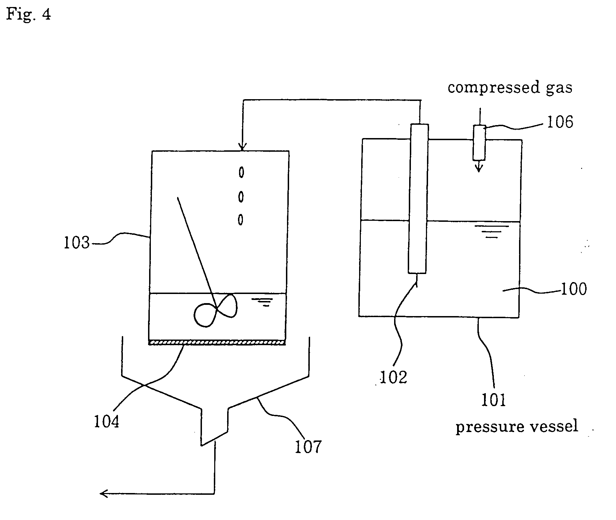

This example as shown in Fig. 4 illustrates a case where a sample liquid is concentrated using a filter impervious to bacteria.

More specifically, a predetermined amount of a sample liquid 100 to be subjected to detection of, for example, whether or not a given bacterium exists therein is sampled and filled in a hermetically sealed pressure vessel 101, and a compressed gas (e.g., compressed air, compressed nitrogen, or the like) is then allowed to work on the sample liquid 100 in the pressure vessel 101 to transfer the sample liquid 100 little by little through a capillary 102 to a next-stage pressure vessel 103, wherein water is then filtered through a microfilter 104 in the next-stage pressure vessel 103 to prepare a concentrate 105 containing bacteria concentrated therein. The resultant liquid becomes a water sample wherein a bacterium is concentrated. A phage solution is added to this bacterium-containing concentrate 105 from which the bacterium is then detected using, for example, a microscope or a fluorescence microscope. Additionally stated, in Fig. 4, numeral 106 refers to a nozzle for feeding the compressed gas to the pressure vessel 101, and 107 to a drainage system for the liquid passed through the microfilter 104.

According to this example, a phage solution can be added to a sample liquid wherein a bacterium is concentrated by membrane filtration, whereby chances of contact of the bacterium with the phage, which is a matter of importance in the present invention, can be realized with a high probability.

### Embodiment 7

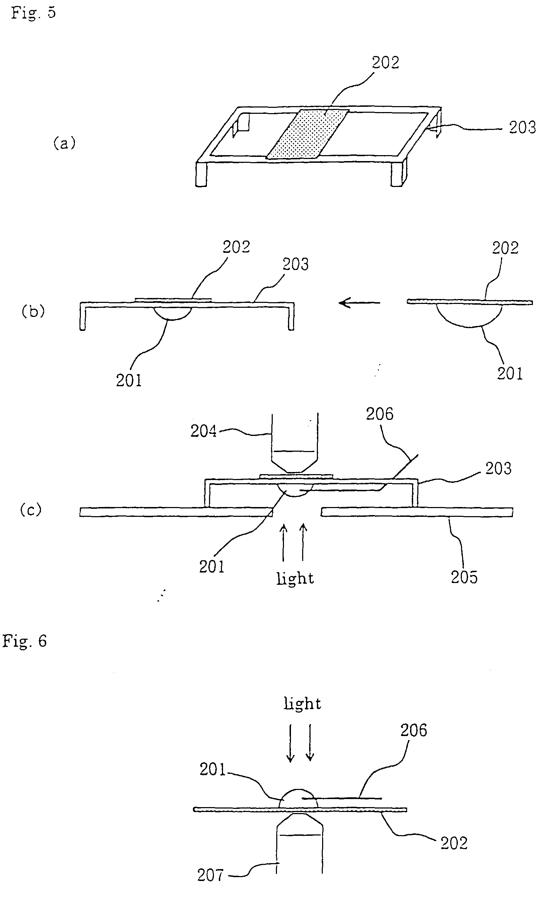

This example as shown in Fig. 5 illustrates a case where a bacterium is concentrated in a water sample while using an attractant to enhance the sensitivity of detection.

In this example, a dropwise water sample having nutrient salts added thereto is attached to the lower surface of a slide glass 202 (see Fig. 5 (b)), which is then set on a slide glass holder 203, which is then placed on the stage 205 of, for example, a microscope. When a bacterium is detected through the lens 204 of the microscope, a solution containing an attractant such as serine, aspartic acid, glucose or galactose, and a phage are fed to the site of the focus of the lens via the tip of a capillary 206 extending along the slide glass 203 from one side thereof. Additionally stated, a surface of the slide glass to which the water sample is attached may desirably be coarsened to enable the amount of the attached water sample to be increased.

According to the foregoing procedure, the bacterium converges upon the site of injection of the solution containing the attractant (the site of the focus of the lens) wherein the phage fed together with the attractant is adsorbed on the bacterium into which the phage DNA labeled with a dye or a fluorochrome is then injected. Thus, a state in which the bacterium converges near the tip of the capillary can be observed.

Additionally stated, in this example, the water sample 201 is dropwise attached to the lower surface of the slide glass 202 because the water sample is desired to be observed in an open state rather than a state in which the water sample is covered with a cover glass according to a method of observing a water sample dropped on a slide glass, thereby increasing the tendency of a bacterium to be attracted to ambient air rather than to an attractant because of prompt deficiency of O₂ in the water sample. Additionally stated, the speed of attraction of the bacterium by the attractant is so very high that drying of the water sample does not pose a problem.

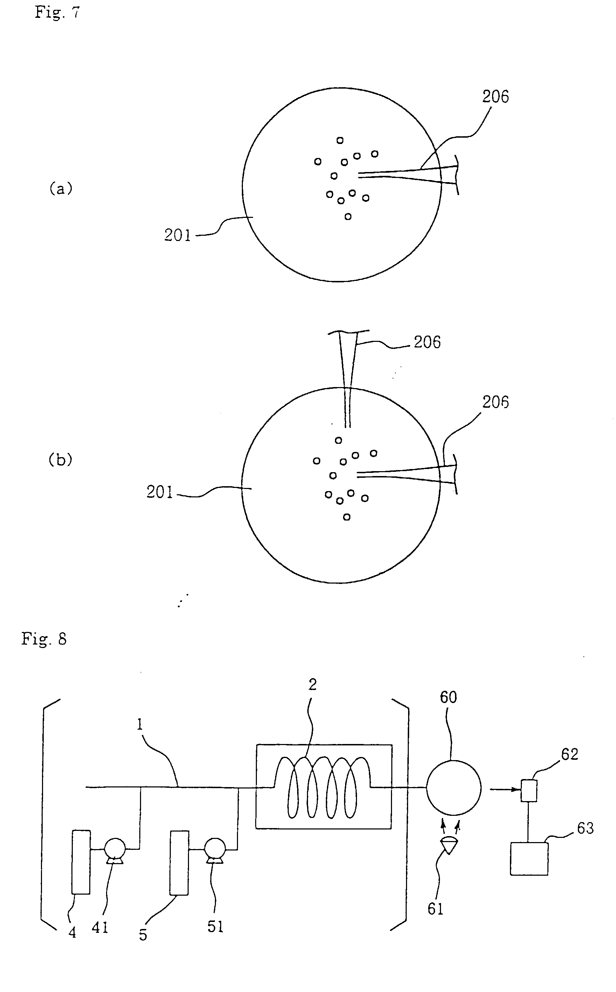

Although the phage is fed together with the attractant by the single capillary 206 as shown in Fig. 7 (a) in this example, the phage solution may alternatively be fed after microscopic confirmation of convergence of the bacterium by feeding thereto the attractant while using two capillaries as shown in Fig. 7 (b).

### Embodiment 8

Fig. 6 illustrates a case where an attractant and a phage solution are fed by means of a capillary 206 to a water sample 201 attached to the upper surface of a slide glass 202 while using an inverted microscope. Numeral 207 refers to the lens of the inverted microscope.

Since this case is a method wherein the water sample is attached to the upper surface of the slide glass 202, it is advantageous in that a comparatively large amount of the water sample can be attached thereto.

### Embodiment 9

This example illustrates a case where the water sample (sample liquid) passed through the line 1, for example, as shown in Fig. 1 to 3 is a water sample prepared by diluting a solution thickly containing a bacterium at a predetermined dilution ratio in the range of a few tens to several thousands, so that substantially the same procedure of bacterium detection as in the foregoing Embodiments can be adopted.

According to this example, the state of the activity of a useful microorganism can be controlled in production facilities or the like, for example, for obtaining a given product by making the most of the metabolic activity of the microorganism (bacterium).

### Embodiment 10

This example illustrates a case where a sample liquid 60 obtained, for example, by the same treatment as in Fig. 1 is irradiated with a light from a light source in the form of a fluorochrome-exciting light source 61, for example, as shown in Fig. 8, while a fluorescence emitted by a bacterium having a fluorochrome-labeled nucleic acid injected thereinto from a phage is imagewise picked up with an image pickup tube device 62 provided, for example, in such a way as to have a function of a fluorescence microscope to obtain picked-up image information, which is then subjected to predetermined image processing with an image processing unit (generally a computer) 63 to mechanically and automatically detect the bacterium stained with the fluorochrome.

Additionally stated, the method of this example is not limited to the foregoing detection method involving fluorescence detection, but may be carried out according to a procedure differing in labeling substance and/or detection method, some examples of which will be mentioned below.
(1) An image picked up with a CCD camera or the like is resolved in the x and y directions at a predetermined resolution to detect pixels (image elements) with distinction of fluorescent points from the background while classifying the luminance of every pixel for the fluorescent points, for example, according to gradations of 0 to 255 for general use in the gradation analysis method to compare it with a predetermined threshold value of luminance, whereby light sources in excess of the threshold value are recognized as a bacterium. In this case, continuous light source pixels in excess of the threshold value are each judged as a single bacterial cell.
(2) An image picked up with a CCD camera or the like is resolved in the x and y directions at a predetermined resolution to detect pixels (image elements) with distinction of fluorescent points from the background while computing the continuous length in x or y direction or area of every pixel detected as a fluorescent point to compare the computation with a predetermined threshold value of length or area for detection of a bacterium.
(3) A spectral image of a given hue picked up with a CCD camera or the like is resolved in the x and y directions at a predetermined resolution to detect pixels (image elements) with distinction of dye-detected points from the background while computing the continuous length in x or y direction or area of every pixel detected as a dye-detected point to compare the computation with a predetermined threshold value of length or area for detection of a bacterium.
(4) Image processing is carried out in such a way that pixels in excess of the threshold value among those for fluorescence-detected points or dye-detected points as described in (1) to (3) above are left as detected points while canceling pixels of at most the threshold value regarded as the background (rendering the levels thereof to that of the background).

Additionally stated, although the example of Fig. 8 illustrates only the fluorochrome-exciting light source 61 and the image pickup tube device 62 as constituting an optical detection means, it is a matter of course that other lens system, filter, etc. may be provided as needed. Further, the foregoing image processing or the like can be carried out using a known computer or an image processing technology.

### EXAMPLES

### Referential Example 1

In order to confirm that the intensity of fluorescence is increased in a state of a phage nucleic acid being diffused after burst from phage particles as against a state of the phage nucleic acid being packed in the phage particles while using an *Escherichia coli*-specific phage T4 having the nucleic acid thereof labeled with a fluorochrome and prepared in the following Phage Preparation Example (1-1), the following tests were carried out.

Specifically, the intensity of fluorescence was measured for each of Sample I which was a phage solution having a concentration of 3x10⁸/ml (a solution of a phage having the fluorochrome-labeled nucleic acid) and Sample II which was a solution of phage particles destroyed by treating them with a surfactant and an alkali.

As a result, the fluorescence intensity of the solution of Sample I was 24.4, whereas the fluorescence intensity of the solution of Sample II was 52.3. Thus, it is understandable that a fluorochrome-labeled phage nucleic acid is increased at least twofold in fluorescence intensity through diffusion thereof.

### Referential Example 2

*Escherichia coli* was suspended in each of physiological saline and a nutrient medium to provide a concentration of 10⁸/ml. On the other hand, *Pseudomonas* was separately suspended in a nutrient medium to provide the same concentration.

Subsequently, the same *Escherichia coli*-specific phage T4 having the fluorochrome-labeled nucleic acid as used in Referential Example 1 was added to each of the resulting preparations to provide a concentration of 3x10⁹/ml. The resulting mixture was allowed to stand at 37 °C for 10 minutes, followed by measurement of the fluorescence intensity thereof.

As a result, it was found out that the fluorescence intensity of the *Escherichia coli* sample suspended in physiological saline was 25.2, that the fluorescence intensity of the *Escherichia coli* sample suspended in the nutrient medium was 119.7, and that the fluorescence intensity of the *Pseudomonas* sample suspended in the nutrient medium was 28.7.

It is seen from the foregoing that, in the *Escherichia coli* sample suspended in physiological saline, the phage nucleic acid was not injected into *Escherichia coli* despite adsorption of the phage on *Escherichia coli* because of insufficiency of nutrients for *Escherichia coli* (insufficiency of metabolizable energy), whereby no increase in fluorescence intensity was recognized with substantially the same value of fluorescence intensity as in Referential Example 1.

On the other hand, in the *Escherichia coli* sample suspended in the nutrient medium, adsorption of phage - injection of phage nucleic acid into *Escherichia coli* (burst-diffusion) occurred, whereby a remarkable increase in fluorescence intensity was recognized.

By contrast, it is seen that, in the *Pseudomonas* sample suspended in the nutrient medium, no significant increase in fluorescence intensity was recognized because neither adsorption of the *Escherichia coli*-specific phage T4 on *Pseudomonas* nor burst of the phage nucleic acid occurred.

It is seen from the foregoing results that the existence or nonexistence of a bacterium specific for a phage can be simply and surely judged by bringing the bacterium-specific phage having the fluorochrome-labeled nucleic acid into contact with the bacterium having a metabolic activity in the presence of a given nutrient source, and subsequently measuring the intensity of fluorescence.

It is also seen that a given bacterium can be quantitatively determined using a calibration curve or the like with preliminary confirmation of the fluorescence intensity-versus-cell count relationship.

### (1) Example 1. Detection of Escherichia coli with Bacteriophage T4

### (1-1) Preparation of Phage Having Fluorochrome-Labeled Nucleic Acid

*Escherichia coli* strain B was aerobically cultivated in an L broth medium (containing 10 g of Bacto-Tryptone, 5 g of yeast extract and 5 g of sodium chloride per liter) at 37 °C, and bacteriophage T4 (No. 20004) procured from IFO (Institution for fermentation, Osaka) was then added at the metaphase of logarithmic growth phase to propagate the phage. The medium for use in propagational cultivation of the phage is not particularly limited inso far as it is a nutrient-rich medium.

In order to prepare phage particles having the DNA thereof labeled with a fluorochrome, the phage was added to the medium, to which 4,6-diamidino-2-phenylindole hydrochloride (DAPI) as a fluorochrome was simultaneously added in an amount of 1 mg per liter of the medium. Additionally stated, the fluorochrome is not particularly limited in so far as it is a substance having an affinity for the phage DNA.

Aerobic cultivation was continued at 37 °C till the phage propagated in cells of *Escherichia coli* to burst into the medium through bacteriolysis. Thereafter, the culture medium containing the phage was centrifugalized at a low speed (3,000 rpm, 10 minutes) to remove the cell residue. Sodium chloride was added and dissolved to provide a concentration of 1 M, and polyethylene glycol was further added and dissolved to provide a concentration of 10 %. The resulting mixture was allowed to stand at 4 °C for one night, after which the resulting agglutination product was recovered by centrifugalization (15,000 rpm, 20 minutes). This precipitate was suspended in a small amount of a 20 mM tris hydrochloride buffer containing 5 mM of magnesium sulfate. The resulting suspension was admixed with the same amount of chloroform, vigorously stirred for 30 seconds, and then centrifugalized to be separated into a chloroform layer and a water layer. The water layer containing the phage was collected and centrifugalized at 30,000 rpm for 30 minutes to recover the phage. The resulting precipitate was suspended in a small amount of a 5 mM magnesium sulfate/20 mM tris hydrochloride buffer.

This suspension was placed on a discontinuous density gradient with cesium chloride densities of 57 %, 46 % and 33 %, and centrifugalized with a horizontal rotor at 30,000 rpm at 4 °C for 3 hours. The formed phage band was collected and dialyzed to remove cesium chloride, whereby a refined preparation of fluorochrome-labeled phage was obtained. The concentration of the obtained refined preparation of the phage was 10¹² particles/ml.

### (1-2) Detection of Escherichia coli

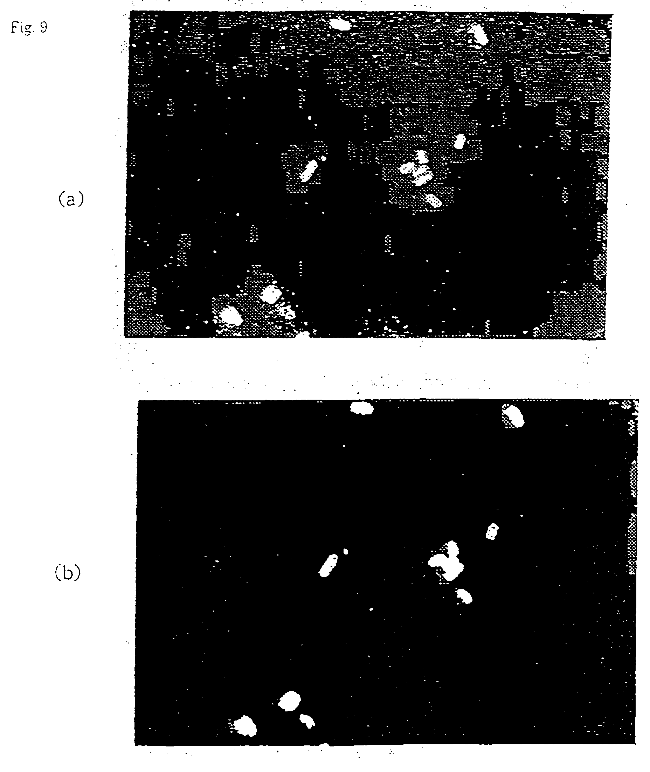

2 µl of the above-mentioned refined preparation of fluorochrome-labeled phage was added to 1 ml of a solution containing cultivated *Escherichia coli* (*Escherichia coli* concentration: 10⁸ cells/ml), and then observed with a fluorescence microscope to confirm the fluorochome-stained cells of *Escherichia coli*. Additionally stated, Fig. 9 (a) shows a microscopic photograph of a fluorescent image of the *Escherichia coli*-containing solution having the phage added thereto, which image was picked up with an epifluorescence microscope BH2-RFL (manufactured by Olympus Optical Co., Ltd.), while Fig. 9 (b) shows the results of image processing for canceling fluorescent points of at most a predetermined threshold value by computing continuous lengths of fluorescent points in the x or y direction in this image.

By contrast, cells of other bacterial strains, even if admixed with the above-mentioned refined preparation of fluorochrome-labeled phage, were not stained fluorescent. It would be confirmable from the foregoing that recognition of a host by a phage is strict.

On the other hand, no fluorescence was recognized when *Escherichia coli* was sterilized with chlorine and then admixed with the above-mentioned preparation of fluorochrome-labeled phage. It was confirmed from this result that extinct *Escherichia coli* (dead *Escherichia coli*) was not stained fluorescent, and that a phage DNA was injected only into viable cells.

It was confirmed from the foregoing results that only cells of *Escherichia coli* having a biological activity could be specifically detected using an *Escherichia coli*-specific phage having the fluorochrome-labeled nucleic acid according to the method of the present invention.

### (2) Example 2. Measurement of Cell Count of Escherichia coli with Flow Cytometer

The fluorochrome-labeled Phage T4 obtained in Example 1 (1-1), diluted with a solution of 20 mM tris hydrochloride (Tris-HCl) and 5 mM MgSO₄ having a pH of 7.5 to provide a concentration of 10¹⁰ particles/ml, was measured with a flow cytometer (BRYTE HS manufactured by Biorad Company) using flow cytophotometry, but fluorescent particles were hardly detected.

By contrast, when the test of measurement with the above-mentioned flow cytometer was carried out under substantially the same conditions except that *Escherichia coli* as the host of the phage was added to the phage solution containing the same number of phage particles, many fluorescent particles were detected in a high fluorescence intensity region.

It is apparent from the results of the two tests of measurement without addition of any fluorochromes other than phage particles that particles high in fluorescence intensity, which particles appeared when *Escherichia coli* was added afresh, were derived from *Escherichia coli* stained fluorescent by the phage DNA injected thereinto with the aid of the bacteriophage.

Additionally stated, the reason for detection of no fluorescence from the phage solution not containing *Escherichia coli* added thereto with the above-mentioned device is believed to be that the intensity of fluorescence was below the limit of detection with the above-mentioned device due to the extreme fineness of the phage particles having the phage DNA or due to the morphology of the phage DNA existing in the phage, though which is the true cause is not necessarily apparent. Accordingly, optical means and information processing means of a system capable of not only detecting a fluorochrome-labeled DNA in a phage as well as in a bacterium but also distinguishing a difference between the states of the two cases by a difference in fluorescence intensity therebetween can be used though it depends on the kind of optical detector to be used.

Further, in order to confirm the foregoing fluorescence-based detection of *Escherichia coli* with the aid of a phage DNA, *Escherichia coli* was given a fluorescence directly by a fluorochrome without using a phage to measure the intensity thereof. Additionally stated, for directly staining *Escherichia coli* fluorescent, 1 µg/ml of DAPI was added to cultivated *Escherichia coli* to stain the DNA of *Escherichia coli*.

As a result, substantially the same number of particles showing the same intensity of fluorescence as that of fluorescence given with the aid of the phage was detected.

As described above, substantially the same level of fluorescence detection as in the conventional method wherein *Escherichia coli* is labeled directly with a fluorochrome is possible according to the method of the present invention. Furthermore, staining a bacterium fluorescent with the aid of a phage according to the present invention can provide an advantage that only the strictly chosen specific bacterium can be specifically stained unlike according to the method of directly staining a bacterium with a fluorochrome, whereby the quantitative determinability of the bacterium can be remarkably improved.

### (3) Example 3. Selective Detection of Escherichia coli with Bacteriophage T4

In this Example, there were carried out tests on whether or not enterobacteria isolated from environment can be identified with the aid of bacteriophage T4.

Sewerage in Osaka-fu as a water sample was spread over a desoxycholate agar plate (Eiken Chemical Co., Ltd.). In this agar plate, *Escherichia coli* and bacteria included in coliform bacteria form red colonies, whereby they can be distinguished from other general bacteria.

Among colonies formed on the agar plate coated directly with sewerage, 50 colonies were picked up from red colonies believed to contain Escherichia coli or any coliform bacterium, and examined with respect to exhibition of fluorescence with the aid of bacteriophage T4 labeled with DAPI and activity of β-glucuronidase. β-Glucuronidase is an enzyme specific for *Escherichia coli*. *Escherichia coli* can be distinguished from coliform bacteria on the basis of the existence or nonexistence of this enzyme (city water test method; specific enzyme-substrate culture medium method).

Further, the bacterial species were simultaneously identified according to Code in Biotest No. 1 (Eiken Chemical Co., Ltd.). The results are shown in Table 1 below. Table 1 shows the results of identification of *Escherichia coli* and bacteria believed to be those included in coliform bacteria.

**Table 1**

| Tests on Bacteria Isolated from Sewerage (Bacteria That Formed Red Colonies) | | | | |
|---|---|---|---|---|
| No. | Biotest Code | Bacterial Species | β-Glucuronidase | Fluorochrome-Labeled Phage |
| 1 | 0611517 | | - | + |
| 2 | 0241407 | E. coli | - | ± |
| 3 | 0651517 | | - | + |
| 4 | 0211517 | E. coli | - | + |
| 5 | 0201537 | E. coli* | - | + |
| 6 | 0241407 | E. coli | - | ± |
| 7 | 0211517 | E. coli | - | + |
| 8 | 0201507 | E. coli | - | + |
| 9 | 0201507 | E. coli*;Shigella sp. | - | + |
| 10 | 0251527 | | - | + |
| 11 | 0251407 | | - | + |
| 12 | 0201537 | E. coli* | - | + |
| 13 | 0611517 | E. coli* | - | + |
| 14 | 0001517 | Serratia p. | - | + |
| 15 | 1241407 | E. coli | - | + |
| 16 | 0211517 | E. coli | - | + |
| 17 | 0241407 | E. coli | - | + |
| 18 | 0211517 | E. coli | - | + |
| 19 | 0201517 | E. coli*;Shigella sp. | - | + |
| 20 | 0201517 | E. coli*;Shigella sp. | - | + |
| 21 | 6135535 | Citrobacter | - | - |
| 22 | 4117377 | | - | - |
| 23 | 0101507 | Enterobacter**;Serratia* | - | + |
| 24 | 0241417 | E. coli | - | + |
| 25 | 0241407 | E. coli | - | + |
| 26 | 0201517 | E. coli*;Shigella sp. | - | + |
| 27 | 0201517 | E. coli*;Shigella sp. | - | ± |
| 28 | 0201407 | E. coli | - | + |
| 29 | 0251517 | | - | + |
| 30 | 0201517 | E. coli*;Shigella sp. | - | + |
| 31 | 0347777 | Klebsiella ox. | - | + |
| 32 | 0757777 | | - | - |
| 33 | 2261535 | E. coli*** | + | + |
| 34 | 0201517 | E. coli*;Shigella sp. | - | ± |
| 35 | 0201517 | E. coli* | - | ± |
| 36 | 2271535 | E. coli* | + | ± |
| 37 | 0201407 | E. coli | - | + |
| 38 | 0201407 | E. coli | - | - |
| 39 | 2261537 | E. coli*** | + | + |
| 40 | 2271535 | E. coli | + | - |
| 41 | 6105577 | Citrobacter | - | + |
| 42 | 2261537 | E. coli*** | + | + |
| 43 | 2107777 | Klebsiella | - | - |
| 44 | 2201735 | Enterobacter | + | - |
| 45 | 2261537 | E. coli*** | + | + |
| 46 | 2271535 | E. coli* | + | + |
| 47 | 4101577 | Citrobacter | - | + |
| 48 | 0241417 | E. coli | - | ± |
| 49 | 0271535 | E. coli | - | - |
| 50 | 0347777 | Klebsiella | - | ± |

Additionally stated, in Table 1, "+" indicates occurrence of an active reaction (positive), and "-" indicates no occurrence of any active reaction (negative) On the other hand, blanks in "Bacterial Species" column mean that no bacteria corresponding to Code in Biotest were found. The asterisk '*' after the names of bacterial species is used to indicate a rate of conformity of at least 10 % for bacterial species denoted by ***, a rate of conformity of at least 1 % for one denoted by **, a rate of conformity of at least 0.2 % for those denoted by *, and a rate of conformity of less than 0.2 % for those with no such symbols.

The foregoing results in Table 1 shows that the rate of conformity was 16 % between the method using any active reaction of β-glucuronidase and Biotest, whereas the rate of conformity was 94 % between the method of the present invention and Biotest.

This confirms that the method of the present invention is markedly higher in the level of quantitative determination than the conventional method.

### (4) Example 4. Concentration of Bacterium by Attractant

There were prepared 0.1 ml of a water sample containing cultivated *Escherichia coli* (*Escherichia coli* concentration: 10⁸ cells/ml), the refined preparation of bacteriophage T4 having the fluorochrome-labeled nucleic acid and prepared in Example 2, and an attractant solution containing 1 mM of serine as an attractant. According to Embodiment 7, the water sample was attached to the lower surface of a slide glass, which was then set in a fluorescence microscope. The refined preparation of Phage T4 and the attractant solution were injected into the water sample via a capillary or capillaries (capillary or capillaries formed by heating a glass tube with a burner and extending it) to observe the water sample with the fluorescence microscope.

The results of observation are shown in Fig. 7. Fig. 7 (a) shows the results in the case where the refined preparation of Phage T4 and the attractant solution were injected into the water sample via a single capillary after they were mixed with each other, while Fig. 7 (b) shows the results in the case where the refined preparation of phage T4 and the attractant solution were injected into the water sample via separate capillaries. As is understandable from these results, the bacterium gathered near the tip(s) of the capillary (capillaries), whereby it was confirmed that high-sensitivity detection was possible.

### (5) Example 5. Simultaneous Detection of 2 Bacteria with 2 Phages Labeled with 2 Fluorochromes Differing in Wavelength

### (5-1) Preparation of Phage Having Fluorochrome-Labeled Nucleic Acid

*Escherichia coli*-specific phage T4 labeled with Acridine Orange was prepared according to substantially the same procedure as the procedure of phage preparation in Example 1 (1-1) above except that Acridine Orange as a fluorochrome was used instead of DAPI. On the other hand, *Salmonella*-specific phage P22 labeled with DAPI was prepared according to substantially the same procedure as the procedure of phage preparation in Example 1 (1-1) above except that *Salmonella*-specific phage P22 (No. 20023) procured from IFO was used as a phage.

The above-mentioned *Escherichia coli*-specific phage T4 labeled with Acridine Orange was observed as yellowish green fluorescent particles when observed at an excitation wavelength of 492 nm with a fluorescence microscope, while the *Salmonella*-specific phage P22 labeled with DAPI was observed as blue fluorescent particles when observed at an excitation wavelength of 345 nm. Further, when these phages were observed after they were mixed with each other, respective observations thereof were not mutually interfered since the excitation wavelengths and fluorescence wavelengths of the respective fluorochromes were different from each other.

### (5-2) Detection of Escherichia coli and Salmonella

A nutrient broth having a cultivated *Escherichia coli* concentration of 10⁹ cells/ml was mixed with the same amount of a nutrient broth having a cultivated *Salmonella* concentration of 10⁹ cells/ml to prepare a test liquid, to which *Escherichia coli*-specific phage T4 labeled with Acridine Orange and *Salmonella*-specific phage P22 labeled with DAPI were added to provide respective concentrations of 10¹⁰ particles/ml. The resulting mixture was allowed to stand at 37 °C for 10 minutes, and then observed with a fluorescence microscope to confirm *Escherichia coli* dyed with the Acridine Orange-labeled phage T4 at an excitation wavelength of 492 nm and *Salmonella* stained with the DAPI-labeled phage P22 at an excitation wavelength of 345 nm, followed by counting the number of the cells of each bacterium in 10 fields of vision. The cell counts of *Escherichia coli* recognized at an excitation wavelength of 492 nm were 20, 15, 27, 22, 28, 42, 30, 18, 22, and 22 with 24.6 on the average. On the other hand, the cell counts of *Salmonella* recognized at an excitation wavelength of 345 nm were 19, 33, 27, 35, 38, 14, 48, 25, 18, and 22 with 27.9 on the average. Thus, the observed cell counts were in good agreement with the ratio in the original test liquid prepared by mixing them at a cell count ratio of 1:1.

It was confirmed from the foregoing results that use of 2 (or more) phages stained with respective 2 (or more) fluorochromes differing in wavelength (excitation wavelength or fluorescence wavelength) enables 2 (or more) given bacteria to be simultaneously detected.

### (6) Example 6. Measurement of Total Number of 2 Bacteria with 2 Phages Stained with the Same Fluorochrome

### (6-1) Preparation of Phage Having Fluorochrome-Labeled Nucleic Acid

*Escherichia coli*-specific phage T4 labeled with DAPI and prepared in Example 1 (1-1), and the above-mentioned *Salmonella*-specific phage P22 labeled with DAPI and prepared in Example 5 (5-1) were used as phages having the respective fluorochrome-labeled nucleic acids.

### (6-2) Detection of Escherichia coli and Salmonella

A nutrient broth having a cultivated *Escherichia coli* concentration of 10⁸ cells/ml was mixed with the same amount of a nutrient broth having a cultivated *Salmonella* concentration of 10⁸ cells/ml to prepare a test liquid, to which the fluorochrome-labeled phage T4 and phage P22 were added to provide respective concentrations of 10¹⁰ particles/ml. The resulting mixture was allowed to stand at 37 °C for 10 minutes, and the cell count was then measured by flow cytometry. The measured value of the number of fluorescent particles was 8x10⁷/ml.

By contrast, when the cell count of a liquid prepared by mixing the *Escherichia coli*-containing culture medium with the same amount of a *Salmonella*-free culture medium was measured in the same manner by flow cytometry, the measured value of the number of fluorescent particles was 3x10⁷/ml. On the other hand, when the cell count of a liquid prepared by mixing the *Salmonella*-containing culture medium with the same amount of an *Escherichia coli*-free culture medium was measured in the same manner by flow cytometry, the measured value of the number of fluorescent particles was 3x10⁷/ml. These values, which were about half of the measured value of the cell count of the test liquid containing both of the above-mentioned bacteria, were in good conformity therewith.

It is understandable from these results that the total cell count of 2 or more given bacteria can be found by labeling 2 or more phages with the same dye.

### (7) Example 7. Influences of Added Phage Concentration on Results of Detection

A test was carried out for the purpose of confirming whether or not a change in added phage concentration exerts influences on the cell count of a bacterium to be detected and the time required to make detection possible.
(7-1) 10⁶ cells/ml of *Escherichia coli* in L broth were prepared. The fluorochrome-labeled phage T4 obtained in Example 1 (1-1) was added to this cultivated *Escherichia coli*-containing solution to provide phage concentrations of 10⁴ particles/ml, 10⁸ particles/ml, 10⁹ particles/ml, and 10¹⁰ particles/ml.
(7-2) Incubation was carried out at 37 °C, and a given amount (60 µl) of each sample was sampled at given intervals to determine *Escherichia coli* by flow cytometry.

Markedly fluorescent particles were counted 15 minutes after incubation for samples having respective phage concentrations of 10¹⁰ particles/ml and 10⁹ particles/ml, both of which gave a measured value of 10⁶ cells/ml, which was in good agreement with the originally prepared cultivated *Escherichia coli* concentration. In these samples, no more change in measured value was recognized even when the incubation time was extended.

In a sample having a phage concentration of 10⁸ particles/ml, the measured value of the number of markedly fluorescent particles was 10⁵ particles/ml 15 minutes after incubation. This measured value was only about 10 % of the actual value of the cell count. In this sample, however, the number of counted particles was about 10⁶ particles/ml when it was measured 30 minutes after incubation.

As for a sample having a phage concentration of 10⁴ particles/ml, no markedly fluorescent particles could be confirmed not only 15 minutes but also 30 minutes after incubation. No markedly fluorescent particles could be confirmed even when the incubation time was further extended.

The foregoing results suggests that, when the concentration of a phage added to a sample is low, a more time is required for detection, and that the number of undetectable bacterial cells is increased.

### INDUSTRIAL APPLICABILITY

According to the present Invention, if only a bacteriophage having the phage DNA in the form of a nucleic acid labeled with a dye or a fluorochrome is brought into contact with a specific bacterium, the bacterium can be dyed or stained by making the most of properties that the bacteriophage specifically recognizes the host bacterium to inject the DNA only into the host bacterium having a biological activity (viable cells), and that the dyed or stained bacterium can be spectrophotometrically analyzed or detected in terms of fluorescence to optically significantly distinguish the bacteriophage having the phage DNA from the bacterium, there can be obtained an advantage that any operation of physically separating the dyed or stained bacterium from the bacteriophage is unnecessary, whereby only the viable cells of the specific bacterium can be detected and identified through a rapid and simple operation to produce an effect that there can be detected a trace amount of the bacterium, the existence of which is to be avoided, for example, in environmental water, environmental air, foodstuffs, city water, sewerage, and water for use and air in hospitals, precision machine factories, and food production as well as processing factories.

Further, according to the inventions of claims 2 and 16 of the instant application, the phage DNA injected into a bacterium captured on a membrane is inhibited from transcription of genes by bonding thereto of a fluorochrome (label), and hence does not produce a group of enzymes for phage particle replication and bacteriolysis, whereby a state in which the bacterium is stained fluorescent can be maintained stably to produce an effect that high-accuracy detection can be done while being unaffected by the treatment time.

According to the inventions of claims 3 to 6 and 17 to 19 of the instant application, since a bacterium is concentrated for detection, high-accuracy sure detection can be realized.

According to the inventions of claims 7 and 20 of the instant application, there can be produced an effect that they are utilizable in control of the activity of useful bacteria for use in food production factories and pharmaceutical factories.

According to the inventions of claims 8 and 21 of the instant application, there can be produced an effect that the existence or nonexistence of a bacterium or the cell count thereof in a water sample flowing through a flow cell can be continuously detected with identification thereof on real time.

According to the inventions of claims 9 and 23 of the instant application, addition of nutrient salts, if necessary, can maintain a bacterium in a metabolizing state to inject a fluorochrome-labeled phage DNA into the bacterium with certainty.

According to the invention of claim 10 of the instant application, the possibility of contact of a phage with a bacterium can be increased to increase the dyeing density of a dye for a single bacterium to be detected or the intensity of fluorescence emitted from such a single bacterium, whereby an effect of making higher-accuracy detection thereof possible can be produced.

According to the invention of claim 11 of the instant application, a sample liquid containing a plurality of bacteria on which different kinds of phages are respectively adsorbed specifically is admixed with phages specific for the respective bacteria, said phages having the respective nucleic acids thereof labeled with respective fluorochromes differing in excitation wavelength or fluorescence wavelength, whereby that plurality of different bacteria can be separately detected according to each excitation wavelength or each fluorescence wavelength, so that operations of detecting that plurality of different bacteria can be simultaneously carried out.

According to the invention of claim 12 of the instant application, since phages respectively adsorbable specifically on a plurality of different bacteria are labeled with the same fluorochrome, for example, whether or not bacteria to be examined exist in a liquid as the object of examination can be easily examined.

According to the inventions of claims 13, 14 and 22 of the instant application, a bacterium can be clearly distinguished from a phage according to a predetermined threshold value on the basis of an picked-up image. Thus, they can be utilizable in automatic determination by making the most of an image processing technology.

According to the inventions of claims 24 and 25 of the instant application, on-site examination can be efficiently done in the case of outdoor detection work.

According to the inventions of the instant application which can produce the foregoing effects, the bacterial examination of city water, sewerage, environmental water and foodstuffs can be markedly improved.

## Claims

1. A method of detecting a bacterium; characterized by comprising bringing a bacteriophage having the nucleic acid thereof labeled with a dye or a fluorochrome into contact with a host bacterium, and detecting the bacterium having the dye-labeled nucleic acid or fluorochrome-labeled nucleic acid injected thereinto with an optical detection means.

2. A method of detecting a bacterium; characterized by comprising capturing a bacterium contained in air or a liquid by a capture means, bringing a bacteriophage having the nucleic acid thereof labeled with a dye or a fluorochrome, for which said bacterium is a host, into contact with the captured bacterium, and detecting the bacterium having the dye-labeled nucleic acid or fluorochrome-labeled nucleic acid injected thereinto with an optical detection means.

3. A method of detecting a bacterium; characterized by comprising concentrating a bacterium to be detected in a sample liquid thinly containing said bacterium, feeding a bacteriophage having the nucleic acid thereof labeled with a dye or a fluorochrome, for which said bacterium is a host, into the resultant liquid containing said bacterium concentrated therein to effect contact thereof with said host bacterium, and detecting the bacterium having the dye-labeled nucleic acid or fluorochrome-labeled nucleic acid injected thereinto with an optical means.

4. A method of detecting a bacterium as claimed in claim 3; characterized in that said bacterium is concentrated by membrane separation or centrifugalization.

5. A method of detecting a bacterium as claimed in claim 3, characterized in that said bacterium is concentrated according to a method comprising feeding an attractant to the given bacterium to be detected in a limited small zone in said sample liquid.

6. A method of detecting a bacterium as claimed in claim 5; characterized in that said attractant is fed through a capillary.

7. A method of detecting a bacterium; characterized by comprising diluting a sample liquid thickly containing a bacterium to be detected, feeding a bacteriophage having the nucleic acid thereof labeled with a dye or a fluorochrome, for which said bacterium is a host, to the resultant diluted sample liquid containing said bacterium to bring it into contact with said host bacterium, and detecting said bacterium having the dye-labeled nucleic acid or fluorochrome-labeled nucleic acid injected thereinto with an optical means.

8. A method of detecting a bacterium; characterized by comprising bringing a bacteriophage having the nucleic acid thereof labeled with a dye or a fluorochrome into contact with a host bacterium in a sample liquid, and detecting the bacterium having the dye-labeled nucleic acid or fluorochrome-labeled nucleic acid injected thereinto according to flow cytophotometry.

9. A method of detecting a bacterium as claimed in any one of claims 1 to 8; characterized by feeding a nutrient source, necessary for adsorption and nucleic acid injection in the initial stage of infection of said host bacterium with said bacteriophage, to said host bacterium.

10. A method of detecting a bacterium as claimed in any one of claims 1 to 9, characterized in that the fluorochrome-labeled phage concentration is 10⁵ to 10¹⁴ particles/ml in said sample liquid containing said bacterium.

11. A method of detecting a bacterium as claimed in any one of claims 1 to 10; characterized by using a phage reagent comprising different kinds of phages having the respective nucleic acids thereof labeled with respective dyes differing in hue or respective fluorochromes differing in excitation wavelength or fluorescence wavelength, and comprising adding said phage reagent to a sample liquid containing a plurality of bacteria on which said different kinds of phages are respectively adsorbed specifically, whereby said plurality of bacteria are simultaneously detected.

12. A method of detecting a bacterium as claimed in any one of claims 1 to 10; characterized by using a phage reagent comprising different kinds of phages having the respective nucleic acids thereof labeled with the same dye or the same fluorochrome, and comprising adding said phage reagent to a sample liquid containing a plurality of bacteria on which these different kinds of phages are respectively adsorbed specifically, whereby the total cell count of said plurality of bacteria are detected.

13. A method of detecting a bacterium as claimed in any one of claims 1 to 12; characterized by comprising measuring the intensity of fluorescence from or the size of light source of fluorescence from a bacterium having the fluorochrome-labeled nucleic acid injected thereinto, and comparing the measured value thereof with a predetermined threshold value, whereby every light source in excess of the threshold value is regarded as the bacterium detected.

14. A method of detecting a bacterium as claimed in any one of claims 1 to 12; characterized in that the existence or nonexistence of a bacterium or the cell count thereof is detected from an image after image processing as to the intensity of fluorescence or the size of light source thereof measured with an optical detection means according to an image processing procedure of excluding light sources having at most a predetermined threshold value of the intensity of fluorescence or the size of light source thereof.

15. A bacterium detector for use in the methods of claims 1 to 14; characterized by comprising a means for adding a solution containing a bacteriophage having the nucleic acid thereof labeled with a dye or a fluorochrome and having a specific adsorbability on a bacterium as an object of detection to a sample liquid, and an optical detection means for detecting said bacterium having the dye-labeled nucleic acid or fluorochrome-labeled nucleic acid injected thereinto.

16. A bacterium detector for use in the methods as claimed in claims 1 to 14; characterized by comprising a capture means for capturing bacteria contained in air or a liquid on a membrane, a phage solution contact means for bringing a phage solution containing a bacteriophage having the nucleic acid thereof labeled with a dye or a fluorochrome into contact with the captured bacteria, and an optical detection means for detecting a bacterium having the dye-labeled nucleic acid or fluorochrome-labeled nucleic acid injected thereinto.

17. A bacterium detector for use in the methods of 1 to 14; characterized by comprising a means for concentrating a bacterium in a sample liquid, a phage solution feed means for feeding a phage solution containing a bacteriophage having the nucleic acid thereof labeled with a dye or a fluorochrome to the concentrated sample liquid, and an optical detection means for detecting said bacterium having the dye-labeled nucleic acid or fluorochrome-labeled nucleic acid injected thereinto.

18. A bacterium detector as claimed in claim 17, characterized in that said concentration means is a membrane separation unit or a centrifuge.

19. A bacterium detector as claimed in claim 17, characterized in that said concentration means is a means for feeding a bacterium attractant into a limited small zone in said sample liquid.

20. A bacterium detector characterized by comprising a means for diluting a sample liquid thickly containing a bacterium, a phage solution feed means for feeding a phage solution containing a bacteriophage having the nucleic acid thereof labeled with a dye or a fluorochrome to the diluted sample liquid, and an optical detection means for detecting the bacterium having the dye-labeled nucleic acid or fluorochrome-labeled nucleic acid injected thereinto.

21. A bacterium detector characterized by comprising a liquid feed means for continuously passing a sample liquid through a flow cell, a phage solution addition means for adding a phage solution containing a bacteriophage having the nucleic acid thereof labeled with a dye or a fluorochrome to said sample liquid in front of said flow cell, and an optical detection means for detecting the bacterium having the dye-labeled nucleic acid or fluorochrome-labeled nucleic acid injected thereinto in said flow cell.

22. A bacterium detector as claimed in any one of claims 15 to 21, characterized by comprising an image pickup means as said optical detection means and an image processing means for excluding light sources having at most a predetermined threshold value of the intensity of fluorescence or the size of light source from a picked-up image obtained by said image pickup means.

23. A bacterium detector as claimed in any one of claims 15 to 22; characterized by comprising a nutrient source feed means for feeding a nutrient source, necessary for adsorption and nucleic acid injection in the initial stage of infection of said host bacterium with said bacteriophage, to said sample liquid.

24. A portable bacterium detection kit for use in the methods of claim 1 or 14; characterized by comprising a bacterium detection aid containing a carbon source capable of being assimilated by a bacterium as the object of detection, a bacteriophage reagent having the nucleic acid thereof labeled with a dye or a fluorochrome, and a reaction vessel prepared for addition thereto of a sample as the object of measurement.

25. A portable bacterium detection kit as claimed in claim 24; characterized in that said bacterium detection aid contains an adsorption aid for promoting the adsorption of a phage on said bacterium.
